# EUROPÄISCHE PATENTSCHRIFT

(11) **EP 4 452 150 B1**
(45) Veröffentlichungstag und Bekanntmachung des Hinweises auf die Patenterteilung: **11.03.2026**
(21) Anmeldenummer: 22840581.7
(22) Anmeldetag: 15.12.2022
(51) Int. Cl.: A61F 5/01, A61F 5/05

(54) **VERFAHREN ZUM ANLEGEN EINER ORTHESE UND ORTHESE**
PROCEDURE FOR APPLYING AN ORTHOSIS AND ORTHOSIS
PROCÉDURE DE MISE EN PLACE D'UNE ORTHÈSE ET ORTHÈSE

(30) Priorität: 21.12.2021 DE 102021134018
(43) Veröffentlichungstag der Anmeldung: 30.10.2024
(73) Patentinhaber: Ottobock SE & Co. KGaA, 37115 Duderstadt (DE)
(72) Erfinder: HÖRIG, Viktor Gerhard, 37115 Duderstadt (DE); KOPPE, Mario, 37115 Duderstadt (DE); BORNMANN, Jonas, 37115 Duderstadt (DE); GONZALEZ VARGAS, Jose, 37115 Duderstadt (DE); OVERDEVEST, Etienne, 37115 Duderstadt (DE); ZAJC, Johannes, 37115 Duderstadt (DE); SCHIRRMEISTER, Benjamin, 37115 Duderstadt (DE)
(74) Vertreter: Gramm, Lins & Partner Patent- und Rechtsanwälte PartGmbB
(86) Internationale Anmeldenummer: PCT/EP2022/086229
(87) Internationale Veröffentlichungsnummer: WO 2023/117712

(56) Entgegenhaltungen:
- DE-A1- 102019 119 645
- DE-A1- 102019 130 391
- DE-U1- 202017 100 201

## Beschreibung

Die Erfindung betrifft ein Verfahren zum Anlegen einer Orthese, die ein erstes formstabiles Bauteil und ein zweites formstabiles Bauteil aufweist sowie eine entsprechende Orthese.

Orthesen sind aus dem Stand der Technik seit langem bekannt und werden verwendet, um ein Körperteil eines Trägers der Orthese, beispielsweise ein Gelenk oder eine Gliedmaße, zu schützen, zu stützen oder im zur Verfügung stehenden Bewegungsspielraum einzuschränken. Dies ist beispielsweise nach einer medizinischen Behandlung, beispielsweise einer Operation, von Vorteil, um beispielsweise ein Gelenk des Trägers zu schonen und vor einer Überbeanspruchung zu schützen. Die beiden formstabilen Bauteile der Orthese werden dann in der Regel an den beiden Körperteilen des Trägers angeordnet, die durch das zu schonende Gelenk verbunden sind. Handelt es sich dabei beispielsweise um einen Ellenbogen, wird das erste formstabile Bauteil am Oberarm und das zweite formstabile Bauteil am Unterarm positioniert.

Aus der DE 10 2019 119645 A1 und der DE 10 2019 130391 A1 ist jeweils eine orthopädietechnische Einrichtung zum Unterstützen des unteren Rückens des Trägers der Einrichtung bekannt, die ein Oberschenkelelement, das am Oberschenkel des Trägers angeordnet wird, und ein Oberkörperelement zum Anordnen am Oberkörper des Trägers aufweist. Diese sind durch wenigstens ein Gelenk miteinander verbunden und werden zum Anlegen oder Ablegen der Einrichtung nicht voneinander getrennt. Sie verfügen jeweils über einen mechanischen Energiespeicher der gespannt und entspannt werden kann, indem das Oberschenkelelement relativ zum Oberkörperelement bewegt wird, wenn eine formschlüssige Verbindung zwischen zwei Elementen vorhanden ist. Diese Elemente sind Formschlusselemente und werden durch Magnete aufeinander zu bewegt und dadurch in Eingriff gebracht. Das Oberschenkelelement und das Oberkörperelement werden durch sie nicht verbunden oder voneinander gelöst.

Aus der DE 20 2017 100 201 U1, die den nächstliegenden Stand der Technik darstellt, ist eine Sprunggelenksorthese bekannt, bei der ein Unterschenkelteil und ein Fußteil mittels Formschluss- und Magnetelementen aneinander befestigt sind.

In einer anderen Anwendung soll beispielsweise ein Gelenk des Trägers in einer bestimmten Haltung, beispielsweise in einem bestimmten Gelenkwinkel, fixiert werden. Dieser hängt in der Regel von der individuellen Situation des Gelenkes und des Trägers ab und kann somit vom Hersteller der Orthese nicht oder nur schwer vorhergesagt werden. Es hat sich daher im Stand der Technik als vorteilhaft erwiesen, wenn bei einer Orthese für diesen Zweck die beiden formstabilen Bauteile in unterschiedlichen Positionen relativ zueinander angeordnet und befestigt werden können.

Oftmals sind Menschen, die einen Schlaganfall, eine Schädigung des Plexus brachialis oder eine vergleichbare Schädigung der Nervenstrukturen oder Nervenleitung erleiden, in der Motorik der oberen Extremität eingeschränkt oder haben sogar einen Totalausfall. Die Einschränkungen sind zumeist durch die fehlende oder unzureichende Enervierung der benötigten Muskeln begründet. Die Unterstützung der Extremität kann durch Orthesen erfolgen. Das Anlegen einer derartigen mehrteiligen Orthese, die mehrere formstabile Bauteile aufweist, erweist sich daher oftmals als schwierig. Dies ist insbesondere für den Fall zutreffend, dass die verschiedenen formstabilen Bauteile der Orthese an unterschiedlichen Körperteilen, die vorzugsweise durch ein Gelenk des Trägers verbunden sind, positioniert werden sollen. Aber auch für den Fall, dass eine bestimmte Position und Haltung eines Körperteils oder eines Gelenkes des Trägers durch die Orthese ermöglicht oder fixiert werden soll, ist das Anlegen der Orthese für den Träger mit Problemen verbunden. Dies gilt umso mehr, wenn die Position oder Haltung nicht die natürliche Position oder Haltung des Körperteils oder des Gelenkes ist.

Der Erfindung liegt daher die Aufgabe zugrunde, ein Verfahren zum Anlegen einer Orthese vorzuschlagen, das einfach und sicher durchführbar ist und dennoch eine ausreichende Sicherheit und Stabilität der Orthese gewährleistet.

Die Erfindung löst die gestellte Aufgabe durch ein Verfahren zum Anlegen einer Orthese mit einem ersten formstabilen Bauteil, das ein erstes Magnetelement und ein erstes Formschlusselement aufweist, und einem zweiten formstabilen Bauteil, das ein zweites Magnetelement und ein zweites Formschlusselement aufweist, wobei das Verfahren folgende Schritte aufweist:
- Anlegen des ersten Bauteiles an einem Körperteil des Trägers, sodass es sich entlang des Körperteils erstreckt, wobei das erste Bauteil noch nicht mit dem zweiten Bauteil verbunden ist,
- Positionieren des zweiten Bauteils an dem ersten Bauteil,
wobei im angelegten Zustand der Orthese zwischen dem ersten Magnetelement und dem zweiten Magnetelement eine magnetische Anziehungskraft wirkt, und das erste Formschlusselement mit dem zweiten Formschlusselement eine formschlüssige Verbindung bildet, durch die eine Bewegung der beiden Bauteile relativ zueinander verhindert wird, wobei nach dem Positionieren des zweiten Bauteils am ersten Bauteil die formschlüssige Verbindung hervorgerufen wird, indem das erste Formschlusselement und das zweite Formschlusselement miteinander in Eingriff gebracht werden, wobei das erste Formschlusselement und/oder das zweite Formschlusselement relativ zueinander bewegt werden. Ein solches Verfahren zeichnet sich erfindungsgemäß dadurch aus, dass das erste Formschlusselement und/oder das zweite Formschlusselement durch eine Kraft, die von dem ersten Magnetelement und/oder dem zweiten Magnetelement ausgeübt wird, aus einer ersten Position in eine zweite Position gebracht wird, wodurch die formschlüssige Verbindung hergestellt wird.

Zunächst wird folglich das erste Bauteil so an dem Körperteil des Trägers angelegt, dass es sich entlang des Körperteils erstreckt. Das erste Bauteil kann beispielsweise eine Schiene oder eine Schale sein und wird an dem Körperteil des Trägers, beispielsweise mittels Befestigungsgurten, angelegt und befestigt. In diesem Zustand ist das zweite Bauteil noch nicht mit dem ersten Bauteil verbunden, sodass auch beispielsweise eine Position der beiden Bauteile relativ zueinander noch nicht beachtet werden muss. Es ist daher für den Träger der Orthese besonders einfach möglich, dass Bauteil an seinem Körperteil anzuordnen.

Erst danach wird das zweite Bauteil an dem ersten Bauteil positioniert. Dabei wird vorzugsweise insbesondere die Position und/oder Orientierung der beiden Bauteile relativ zueinander eingenommen und festgelegt, die für die Funktionsweise der Orthese notwendig und sinnvoll ist. Dabei muss der Träger der Orthese lediglich das zweite Bauteil festhalten und bewegen, da das erste Bauteil sich bereits an seinem Körperteil befindet. Auch dadurch wird der motorische Aufwand, den das Verfahren erfordert, reduziert.

Im angelegten Zustand der Orthese wirkt zwischen dem ersten Magnetelement und dem zweiten Magnetelement eine magnetische Anziehungskraft, die einer ersten Bewegung der beiden Bauteile relativ zueinander entgegenwirkt. Im einfachsten Fall beinhaltet das erste Magnetelement einen Magneten und das zweite Magnetelement einen Magneten, wobei diese Magneten im angelegten Zustand der Orthese so zueinander positioniert sind, dass der Nordpol des einen Magneten zum Südpol des anderen Magneten zeigt.

Zusätzlich zu dieser magnetischen Anziehungskraft bilden das erste Formschlusselement und das zweite Formschlusselement im angelegten Zustand der Orthese eine formschlüssige Verbindung, durch die eine Bewegung der beiden Bauteile relativ zueinander verhindert wird. Dies bedeutet nicht zwangsläufig, dass jede Bewegung der beiden Bauteile relativ zueinander verhindert wird. Dies ist zwar eine bevorzugte Ausgestaltung, jedoch nicht notwendig. Für viele Anwendungen ist es ausreichend, wenn einzelne Bewegungen, beispielsweise Rotationen um einzelne Rotationsachse oder Verschiebungen in einzelne Verschieberichtungen, durch die formschlüssige Verbindung verhindert werden. Dies bedeutet ebenfalls nicht zwangsläufig, dass nur eine einzige Bewegung, also beispielsweise die Rotation um eine einzige Rotationsachse oder die Verschiebung in eine einzige Verschieberichtung durch die formschlüssige Verbindung verhindert werden. In bevorzugten Ausgestaltungen werden beispielsweise alle Verschiebungen der beiden Bauteile relativ zueinander bis auf die Verschiebungen eine einzige Verschieberichtung verhindert. Dies bedeutet, dass Verschiebungen, also translatorische Bewegungen der beiden Bauteile relativ zueinander nur in dieser einzigen Verschieberichtung möglich sind, in allen anderen Richtungen jedoch durch die formschlüssige Verbindung verhindert werden. In einer vorteilhaften Ausgestaltung werden beispielsweise alle Rotationen der beiden Bauteile relativ zueinander bis auf die Rotation um eine einzige Rotationsachse verhindert. Dies bedeutet, dass eine Rotation der beiden Bauteile relativ zueinander nur um eine einzige Rotationsachse möglich ist. Die übrigen Rotationen um alle anderen Rotationsachsen werden durch die formschlüssige Verbindung verhindert.

Durch die magnetische Anziehungskraft zwischen dem ersten Magnetelement und dem zweiten Magnetelement werden sämtliche Bewegungen der beiden Bauteile relativ zueinander erschwert, bei denen diese magnetische Anziehungskraft überwunden werden muss. Vorzugsweise werden zumindest einige dieser Bewegungen, vorzugsweise alle diese Bewegungen, durch die formschlüssige Verbindung der beiden Formschlusselemente miteinander verhindert.

Bei dem ersten Magnetelement und/oder dem zweiten Magnetelement handelt es sich vorzugsweise um Permanentmagnete. Das erste Magnetelement und/oder das zweite Magnetelement kann auch als Elektromagnet ausgebildet sein. Um eine magnetische Anziehungskraft zu erzeugen, ist es jedoch nicht notwendig, dass sowohl das erste Magnetelement als auch das zweite Magnetelement von sich aus magnetisch sind, also als Permanentmagnet oder Elektromagnet ausgebildet sind. Es ist vielmehr ausreichend, wenn nur eines der Magnetelemente einen Permanentmagneten oder einen Elektromagneten aufweist und das jeweils andere Magnetelement ein magnetisierbares Element, beispielsweise aus einem ferromagnetischen Material, beinhaltet.

Jedes der beiden formstabilen Bauteile verfügt erfindungsgemäß über ein Formschlusselement und ein Magnetelement. Das Formschlusselement und/oder das Magnetelement kann einstückig mit dem Rest des jeweiligen formstabilen Bauteiles oder einem Anteil davon, beispielsweise einem Grundkörper, einer Schale oder einer Schiene ausgebildet sein. Alternativ dazu kann das Formschlusselement und/oder das Magnetelement auch als separates Element ausgebildet sein, das mit dem Rest des jeweiligen formstabilen Elementes oder einem Anteil davon, beispielsweise einem Grundkörper, einer Schale oder einer Schiene verbunden wird. Die formschlüssige Verbindung zwischen dem erste Formschlusselement und dem zweiten Formschlusselement kann beispielsweise hervorgerufen werden, in dem ein Stecker, der eines der beiden Formschlusselement bildet, in eine korrespondierend ausgebildete Buchse eingesteckt wird, die das jeweils andere Formschlusselement bildet. Derartige Verbindungen sind aus dem Stand der Technik hinreichend bekannt, so dass an dieser Stelle auf eine detaillierte Beschreibung verzichtet wird.

In einer Ausführungsform verfügt beispielsweise sowohl das erste formstabile Bauteil als auch das zweite formstabile Bauteil über eine entsprechende Buchse. Diese sind beispielsweise an Anteil davon, beispielsweise einem Grundkörper, einer Schale oder einer Schiene, angeordnet. Um die beiden formstabilen Bauteile miteinander formschlüssig verbinden zu können, wird vorzugsweise ein Adapter verwendet, der zunächst mit einer der beiden Buchsen verbunden wird. Er wird dadurch Teil des jeweiligen formstabilen Bauteils. Er bildet dann das jeweilige Formschlusselement des formstabilen Bauteils, das mit dem Formschlusselement des jeweiligen anderen formstabilen Bauteils zusammenwirken und die formschlüssige Verbindung erzeugen kann.

Das erste Formschlusselement und/oder das zweite Formschlusselement können als separates Element ausgebildet sein, das mit dem Rest des jeweiligen formstabilen Bauteiles oder einem Anteil davon, beispielsweise einem Grundkörper, einer Schale oder einer Schiene, verbindbar oder verbunden ist. Das erste Magnetelement und/oder das zweite Magnetelement können als separates Element ausgebildet sein, das mit dem Rest des jeweiligen formstabilen Bauteiles oder einem Anteil davon, beispielsweise einem Grundkörper, einer Schale oder einer Schiene, verbindbar oder verbunden ist. Das erste Magnetelement und das erste Formschlusselement können gemeinsam als ein separates Bauteil ausgebildet sein. Das zweite Magnetelement und das zweite Formschlusselement können gemeinsam als ein separates Bauteil ausgebildet sein. Diese Bauteile werden als kombinierte Elemente bezeichnet. Das jeweilige kombinierte Element kann mit dem Rest des jeweiligen formstabilen Bauteiles oder einem Anteil davon, beispielsweise einem Grundkörper, einer Schale oder einer Schiene, verbindbar oder verbunden sein.

Vorzugsweise wird das zweite Bauteil beim Positionieren an dem ersten Bauteil zunächst dem ersten Bauteil angenähert, sodass die magnetische Anziehungskraft entsteht. Die Magnetelemente sind dabei vorzugsweise so angeordnet und ausgebildet, dass die magnetische Anziehungskraft das zweite Bauteil und das erste Bauteil in die gewünschte Position relativ zueinander zieht. Sie kann auf diese Weise die Bewegung der beiden Bauteile beim Positionieren relativ zueinander führen und den Träger der Orthese unterstützen, sodass es diesem leichter fällt, die gewünschte und notwendige Position und/oder Orientierung des zweiten Bauteils relativ zum ersten Bauteil zu erreichen. Die magnetische Anziehungskraft zieht folglich das zweite Bauteil zumindest auch in die gewünschte Position. Der Begriff "zumindest auch" bedeutet in diesem Zusammenhang, dass es durchaus weitere Kräfte geben kann, die die beiden Bauteile relativ zueinander positionieren und orientieren, sodass nicht ausschließlich die magnetische Anziehungskraft die beiden Bauteile relativ zueinander bewegt, aber die magnetische Anziehungskraft "zumindest auch" daran beteiligt ist.

Erfindungsgemäß wird erst nach dem Positionieren des zweiten Bauteils am ersten Bauteil die formschlüssige Verbindung hervorgerufen, indem das erste Formschlusselement und das zweite Formschlusselement miteinander in Eingriff gebracht werden. Dies ist insbesondere dann von Vorteil, wenn die Bewegung, durch die die beiden Bauteile relativ zueinander positioniert wurden, in umgekehrter Richtung durch die formschlüssige Verbindung unterbunden werden soll.

Erfindungsgemäß werden das erste Formschlusselement und/oder das zweite Formschlusselement relativ zueinander bewegt, um die beiden Formschlusselemente miteinander in Eingriff bringen zu können. Eine solche Bewegung wenigstens eines der Formschlusselemente kann beispielsweise durch den Träger der Orthese selbst ausgeführt werden, indem beispielsweise Rastelemente oder Klettverschluss-Elemente, Clips-Elemente oder sonstige Formschlusselemente miteinander in Eingriff gebracht werden.

Erfindungsgemäß wird das erste Formschlusselement und/oder das zweite Formschlusselement durch eine Kraft, die von dem ersten Magnetelement und/oder dem zweiten Magnetelement ausgeübt wird, aus einer ersten Position in eine zweite Position gebracht, wodurch die formschlüssige Verbindung hergestellt wird. Dies kann beispielsweise erreicht werden, indem das erste Formschlusselement und/oder das zweite Formschlusselement wenigstens einen Magneten oder ein magnetisierbares Element enthalten, welches durch das magnetische Feld des ersten Magnetelement des und/oder des zweiten Magnetelement des angezogen oder abgestoßen wird.

Dadurch kommt es zu einer Bewegung, die beispielsweise dazu führt, dass die formschlüssige Verbindung entsteht.

In einer konkreten Ausführungsform handelt es sich bei einem der Formschlusselemente um wenigstens einen Metallstift, der entlang seiner Längsrichtung verschiebbar in einer entsprechenden Bohrung in einem der beiden Bauteile angeordnet ist. Das entsprechend andere Formschlusselement ist in Form ähnlicher Bohrungen im anderen Bauteil ausgebildet. Befinden sich die beiden Bauteile nach dem Positionieren des zweiten Bauteils am ersten Bauteil in der gewünschten Position, in der die formschlüssige Verbindung hervorgerufen werden soll, wird der wenigstens eine Metallstift entlang seiner Längsrichtung innerhalb der Bohrung verschoben, bis er sich teilweise in der korrespondierend ausgebildeten Bohrung im anderen Bauteil befindet. Erstreckt sich nun teilweise in den Bohrungen der beiden Bauteile, wodurch alle Bewegungen bis auf die längst Bewegung entlang der Längsrichtung und der Erstreckungsrichtung der Bohrungen verhindert werden. Die Bewegung selbst kann dabei durch separate magnetische Elemente oder vom ersten Magnetelement und/oder vom zweiten Magnetelement hervorgerufen werden.

In einer bevorzugten Ausgestaltung ist das erste Formschlusselement und/oder das zweite Formschlusselement durch ein Betätigungselement betätigbar. Es wird betätigt, um die formschlüssige Verbindung herzustellen. Das entsprechende Formschlusselement kann beispielsweise in Richtung auf die zweite Position, in der die formschlüssige Verbindung hergestellt ist, vorgespannt sein, indem beispielsweise eine Feder oder ein elastisches Element eine entsprechende Kraft auf das Formschlusselement ausübt. Solange das Betätigungselement nicht betätigt wird, kann die entsprechende Kraft jedoch nicht für eine Bewegung des Formschlusselementes sorgen, etwa weil ein Halteelement das Formschlusselement in der ersten Position, in der noch keine formschlüssige Verbindung entsteht, hält. Durch das Betätigen des Betätigungselement wird dieses Halteelement gelöst und die von der Feder oder dem elastischen Element aufgebrachte Kraft bewegt das jeweilige Formschlusselement aus der ersten Position in die zweite Position und bringt so die beiden Formschlusselemente miteinander in Eingriff.

Vorteilhafterweise entsteht die magnetische Anziehungskraft und die formschlüssige Verbindung bereits beim Positionieren des zweiten Bauteils am ersten Bauteil. Dies geschieht beispielsweise dann, wenn beim Positionieren der beiden Bauteile aneinander eine bestimmte Bewegung der Bauteile zueinander ausgeführt werden muss, etwa um die beiden Bauteile entlang eines bestimmten Pfades zueinander zu bewegen. Dazu verfügt beispielsweise eines der beiden Bauteile über einen Vorsprung, der in einer entsprechenden Kulisse, Nut oder sonstiger Vertiefung oder Öffnung des anderen Bauteils entlanggeführt wird. Dadurch wird bereits beim Positionieren eine formschlüssige Verbindung erzeugt, durch die viele, vorzugsweise alle Bewegungen bis auf die Bewegung, die zum Positionieren der Bauteile relativ zueinander nötig ist, verhindert werden. Entlang dieser Bewegung und des dabei zurückgelegten Pfades nimmt vorzugsweise der Abstand zwischen dem ersten Magnetelement und dem zweiten Magnetelement ab, sodass ein Abstand zwischen ungleichnamigen Polen der beiden Magnetelement abnimmt und dadurch die magnetische Anziehungskraft erhöht wird.

Vorzugsweise wird das zweite Bauteil beim Positionieren an dem ersten Bauteil zudem an einem zweiten Körperteil des Trägers angeordnet. In diesem Fall befindet sich nach dem Positionieren das erste Bauteil an einem ersten Körperteil und das zweite Bauteil an einem zweiten Körperteil. Vorzugsweise sind die beiden Körperteile durch ein Gelenk, beispielsweise ein Knöchelgelenk, ein Knie, ein Handgelenk oder einen Ellenbogen, miteinander verbunden, das durch die Orthese überbrückt wird.

In einer bevorzugten Ausgestaltung ist das erste Bauteil in unterschiedlichen Positionen und/oder Orientierungen an dem zweiten Bauteil befestigbar. Vorzugsweise verfügen die Magnetelemente der beiden Bauteile über mehrere einzelne Magnete oder magnetisierbare Elemente, die so aneinander angeordnet und zueinander ausgerichtet sind, dass die zwischen ihnen wirkende Anziehungskraft die Positionen und/oder Orientierungen der beiden Bauteile relativ zueinander bevorzugen, in denen die Bauteile aneinander befestigt werden können. So können beispielsweise die Magnetelemente der beiden Bauteile jeweils über mehrere einzelne Magnete verfügen, die in unterschiedlichen Orientierungen an dem jeweiligen Bauteil angeordnet sind. In einer bevorzugten Ausgestaltung sind die Einzelmagnete nebeneinander, vorzugsweise äquidistant, am jeweiligen Bauteil angeordnet und um jeweils 180° zueinander versetzt. Dies bedeutet, dass die magnetischen Pole zweier benachbarter Einzelmagnete, die in die gleiche Richtung zeigen, ungleichnamig sind. Sind beide Magnetelemente der beiden Bauteile auf diese Weise ausgebildet, entsteht zwischen den Einzelmagneten, die an den beiden Bauteilen positioniert sind, eine anziehende Kraft, wenn jeweils ungleichnamige Pole einander gegenüber angeordnet sind und eine abstoßende Kraft, wenn jeweils gleichnamige Pole einander gegenüber positioniert sind. Entsprechendes ist auch mit einer Verteilung von Einzelmagneten um den Umfang einer Öffnung und den Umfang eines Vorsprunges möglich. Die Öffnung bildet dann einen Teil eines der Bauteile und der Vorsprung einen Teil des anderen Bauteils. Wird nun der Vorsprung in die Öffnung eingeführt, werden die Einzelmagnete einander gegenüber angeordnet. Handelt es sich auch hier um eine alternierende Ausrichtung, kommt es je nach Winkelstellung zwischen dem Vorsprung unter Öffnung zu einer anziehenden Wechselwirkung zwischen jeweils gegenüberliegenden Einzelmagneten oder zu einer abstoßenden Wechselwirkung. Auch auf diese Weise lassen sich Positionen und/oder Orientierungen durch die magnetischen Wechselwirkungen bevorzugen.

Die Erfindung löst die gestellte Aufgabe zudem durch eine Orthese mit einem ersten Bauteil, das ein erstes Magnetelement und ein erstes Formschlusselement aufweist, und einem zweiten Bauteil, das ein zweites Magnetelement und ein zweites Formschlusselement aufweist, wobei die Orthese eingerichtet ist, mit einem hier beschriebenen Verfahren angelegt zu werden.

Vorzugsweise verfügt das erste Magnetelement und/oder das zweite Magnetelement über mehrere Einzelmagnete, die in unterschiedlichen Nord-Süd-Orientierungen an dem jeweiligen Bauteil angeordnet sind. Damit lassen sich die oben beschriebenen Vorteile erreichen.

Besonders bevorzugt verfügt das erste und/oder das zweite Magnetelement über wenigstens ein magnetisierbares Element, das im angelegten Zustand mit einem magnetischen Element des jeweils anderen Bauteils zusammenwirkt.

Vorteilhafterweise ist wenigstens eines der Magnetelemente oder wenigstens ein Einzelmagnet eines der Magnetelemente an einem der Formschlusselemente befestigt. Besonders bevorzugt ist das erste Formschlusselement und/oder das zweite Formschlusselement durch ein Betätigungselement betätigbar. Besonders bevorzugt verfügt das Betätigungselement über wenigstens einen Magneten.

Mit Hilfe der beigefügten Zeichnungen werden nachfolgend einige Ausführungsbeispiele der vorliegenden Erfindung näher erläutert.

Es zeigen:
- Figuren 1 und 2 -: schematische Darstellungen einer Orthese,
- Figuren 3 bis 11 -: schematische Darstellungen unterschiedlicher Formschluss- und Magnetelemente,
- Figuren 12 bis 15 -: unterschiedliche Stadien beim Verbinden zweier Elemente.

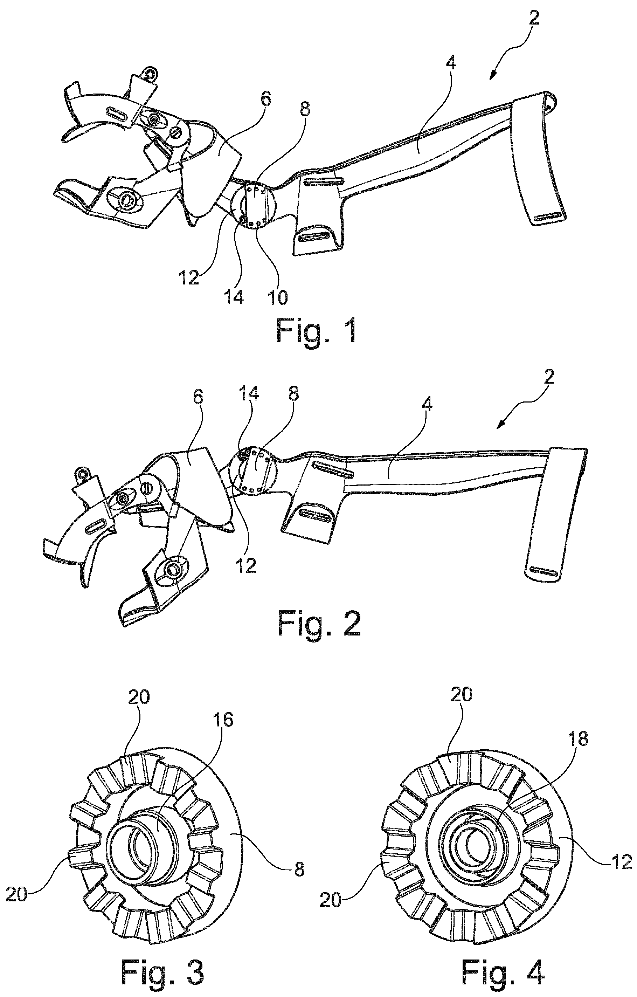

Figur 1 zeigt schematisch eine Orthese 2 für einen Unterarm und eine sich daran befindende Hand. Die Orthese 2 weist ein erstes formstabiles Bauteil 4 zum Anlegen an den Unterarm und ein zweites formstabiles Bauteil 6 zum Anlegen an die Hand auf. Das erste formstabile Bauteil 4 verfügt über ein erstes Formschlusselement 8, dass im gezeigten Ausführungsbeispiel einstückig mit dem Rest des ersten formstabilen Bauteils 4 ausgebildet ist. Im gezeigten Ausführungsbeispiel verfügt das erste Formschlusselement 8 über Ausnehmungen 10, in denen sich erste Magnetelemente befinden, die in der Darstellung der Figur 1 nicht erkennbar sind. Das zweite formstabile Bauteil 6 verfügt über ein zweites Formschlusselement 12, an dem sich zweite Magnetelemente 14 befinden, von denen eines dargestellt ist. In Figur 1 erlauben die Formschlusselemente 8, 12 und die darin enthaltenen oder daran angeordneten Magnetelemente, die beiden formstabilen Bauteile 4, 6 in unterschiedlichen Orientierungen zueinander anzuordnen. Figur 1 zeigt eine erste dieser Orientierungen.

Figur 2 zeigt die gleiche Orthese 2 wie Figur 1. Man erkennt jedoch, dass das erste formstabile Bauteil 4 und das zweite formstabile Bauteil 6 in einer anderen Orientierung relativ zueinander angeordnet sind. Dies ist auch an den beiden Formschlusselementen 8, 12 sowie insbesondere den zweiten Magnetelement 14, von denen in Figur 2 andere erkennbar sind, als dies in der Situation der Figur 1 der Fall war. Das erste formstabile Bauteil 4 und das zweite formstabile Bauteil 6 sind relativ zur Darstellung aus Figur 1 um eine Schwenkachse verschwenkt dargestellt und aneinander befestigt worden, die aus der Zeichenebene herausragt.

Die Figuren 3 und 4 zeigen ein erstes Formschlusselement 8 und ein zweites Formschlusselement 12, die korrespondierend ausgebildet sind. Das in Figur 3 dargestellte erste Formschlusselement 8 verfügt über einen Vorsprung 16, der einen kreisringförmigen Querschnitt aufweist und aus einer Stirnfläche des ersten Formschlusselementes 8 hervorragt. Korrespondierend dazu zeigt das zweite Formschlusselement 12 in Figur 4 eine ringförmige Nut 18, die so ausgebildet ist, dass der Vorsprung 16 des ersten Formschlusselementes 8 in ihr aufgenommen werden kann. Durch die Nut 18 und den sich darin befindenden Vorsprung wird ein Verkippen der beiden Formschlusselemente 8,1 2 relativ zueinander verhindert.

Sowohl das erste Formschlusselement 8 als auch das zweite Formschlusselement 12 verfügen über eine Stirnverzahnung 20, die mehrere über den Umfang verteilte Zähne aufweist. Die beiden Stirnverzahnungen 20 sind korrespondierend zueinander ausgebildet. Werden die beiden Formschlusselemente 8, 12, wie sie in den Figuren 3 und 4 dargestellt sind, miteinander verbunden, wird zunächst der Vorsprung 16 des ersten Formschlusselementes 8 in die dafür vorgesehenen Nut 18 des zweiten Formschlusselementes 12 eingesetzt. Dadurch kommt es zu einer Zentrierung der beiden Formschlusselemente relativ zueinander. In diesem Zustand ist nur noch eine Verdrehung oder Rotation der beiden Formschlusselemente 8, 12 relativ zueinander möglich, wobei die jeweilige Drehachse die Längsachse des Vorsprung 16 ist. Werden die beiden Formschlusselemente 8, 12 weiter aufeinander zu bewegt, kommen die beiden Stirnverzahnungen 20 miteinander in Kontakt und den Eingriff. Ab diesem Moment ist auch eine Rotation um die genannte Drehachse nicht mehr möglich. Dennoch wird durch diese Ausgestaltung erreicht, dass die beiden Formschlusselemente 8, 12 in unterschiedlichen Orientierungen relativ zueinander befestigt werden können.

In einer bevorzugten Ausgestaltung sind an den beiden Formschlusselementen 8, 12 bereits Magnetelemente angeordnet, die in den Figuren 3 und 4 jedoch nicht dargestellt sind. So können beispielsweise Einzelmagnete in den Vertiefungen zwischen jeweils zwei benachbarten Zähnen der Stirnverzahnungen 20 angeordnet werden. Dabei sind unterschiedliche Anordnungen denkbar. Die Einzelmagnete können im ersten Formschlusselement 8 und am zweiten Formschlusselement 12 jeweils gleichgerichtet angeordnet sein. Dies bedeutet, dass die Einzelmagnete am ersten Formschlusselement 8 alle so angeordnet sind, dass ihr Nordpol oder ihr Südpol aus der Stirnfläche herausragt. Analog sind die Einzelmagnete in diesem Fall vorzugsweise am zweiten Formschlusselement 12 so angeordnet, dass ihr Südpol oder ihr Nordpol aus der Stirnfläche herausragt. In diesem Fall wird immer, wenn die beiden Formschlusselemente 8, 12 einander angenähert werden, eine anziehende magnetische Wechselwirkung erzeugt.

Alternativ dazu können die Einzelmagnete in den beiden Formschlusselementen 8, 12 auch alternierend angeordnet sein, sodass ein Einzelmagnete, dessen Nordpol aus der jeweiligen Stirnfläche herausragt von zwei Einzelmagneten benachbart ist, deren Südpol aus der Stirnfläche herausragt. Damit wird nur noch in weniger Orientierungen der beiden Formschlusselemente relativ zueinander eine anziehende Wechselwirkung hervorgerufen.

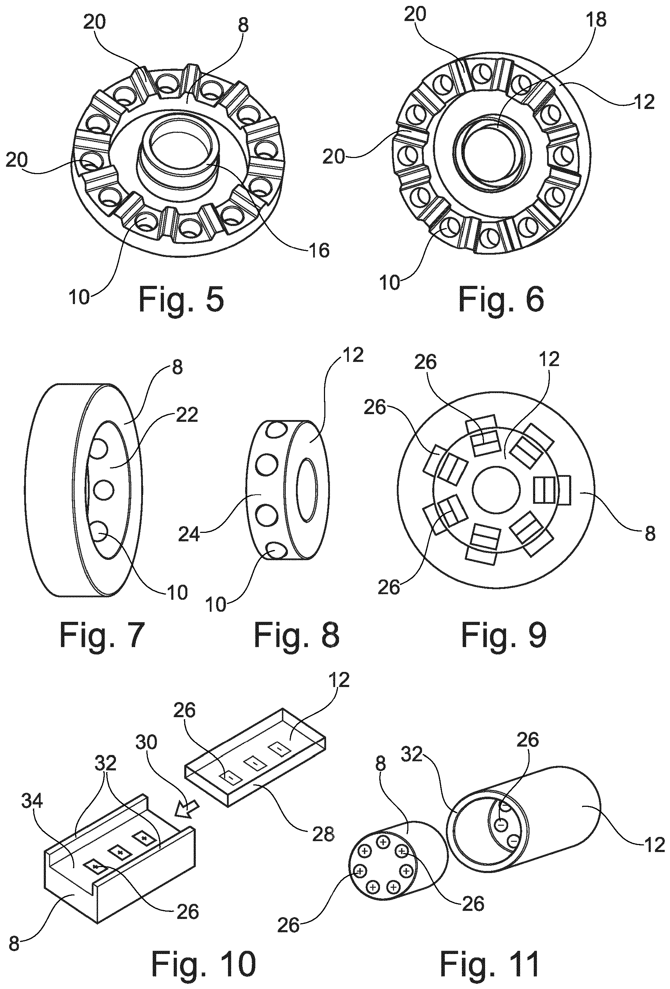

Die Figuren 5 und 6 zeigen ähnliche Formschlusselemente 8, 12 wie die Figuren 3 und 4. Auch hier verfügt das erste Formschlusselement 8 über den Vorsprung 16 und das zweite Formschlusselement 12 über die entsprechende Nut 18. Auch die Stirnverzahnungen 20 sind bei beiden Formschlusselementen 8, 12 vorhanden. Das erste Formschlusselement 8 verfügt über hervorstehende Zähne, während das zweite Formschlusselement 12 über entsprechende Vertiefungen verfügt. Zwischen den jeweiligen Zähnen und Vertiefungen sind Ausnehmungen 10 angeordnet, in denen Einzelmagnete angeordnet werden können.

Die Figuren 7, 8 und 9 eigene schematisch ein weiteres Ausführungsbeispiel der beiden Formschlusselemente 8, 12. Das in Figur 7 dargestellte erste Formschlusselement 8 ist ringförmig ausgebildet und verfügt an seiner Innenseite 22 über die Ausnehmungen 10, in denen die nicht dargestellten Magnetelemente oder Einzelmagnete angeordnet werden können. Der Innendurchmesser des ersten Formschlusselementes 8 entspricht dem Außendurchmesser des in Figur 8 dargestellten zweiten Formschlusselementes 12. Dieses verfügt an seiner Außenseite 24 über die Ausnehmungen 10 für die Einzelmagnete oder Magnetelemente. Wird nun das zweite Formschlusselement 12, wie es in Figur 8 dargestellt ist in das erste Formschlusselement 8 eingeführt, wie es in Figur 7 dargestellt ist, werden die Magnetelemente oder Einzelmagnete, die sich in den Ausnehmungen 10 der beiden Formschlusselemente 8, 12 befinden, einander angenähert und es kommt zu einer magnetischen Wechselwirkung. Ist in Figur 9 schematisch dargestellt. Das zweite Formschlusselement 12 ist innerhalb des ersten Formschlusselementes 8 angeordnet. Schematisch sind Einzelmagnete 26 dargestellt, die in den Ausnehmungen 10 der beiden Formschlusselemente 8, 12 angeordnet und nur so ausgerichtet sind, dass sie miteinander magnetisch wechselwirken.

Figur 10 zeigt eine andere Ausgestaltung der Formschlusselemente. Das zweite Formschlusselement 12 ist als Quader ausgebildet, an dessen Unterseite 28 im gezeigten Ausführungsbeispiel 3 Einzelmagnete 26 angeordnet sind. Das erste Formschlusselement 8 ist so ausgebildet, dass das zweite Formschlusselement 12 entlang des Pfeiles 30 zwischen zwei Begrenzungen 32 eingeschoben werden kann. In dieser Position wird durch die Begrenzungen 32 eine Bewegung der beiden Formschlusselemente 8, 12 quer zur Richtung des Pfeils 30 verhindert, sodass es zu einem teilweisen Formschluss kommt. An einer Oberseite 34 des ersten Formschlusselementes 8 zwischen den beiden Begrenzungen 32 sind ebenfalls Einzelmagnete 26 angeordnet.

In Figur 11 ist das erste Formschlusselement 8 zylinderförmig ausgebildet und verfügt an einer Stirnseite über Einzelmagnete 26, die im gezeigten Ausführungsbeispiel gleichnamig angeordnet sind. Dies bedeutet, dass für alle gezeigten Einzelmagnete 26 der Südpol, gekennzeichnet durch ein "+", aus der Stirnfläche zeigen. Das zweite Formschlusselement 12 verfügt an seiner Stirnfläche über eine ringförmige Begrenzung 32. Der Innendurchmesser dieses Ringes entspricht dem Außendurchmesser des ersten Formschlusselementes 8, sodass dieses um 180° gedreht in die Begrenzung 32 eingeführt werden kann. An der sich darin befindenden Stirnfläche erfüllt auch das zweite Formschlusselement 12 über Einzelmagnete 26, die untereinander gleichnamig angeordnet sind.

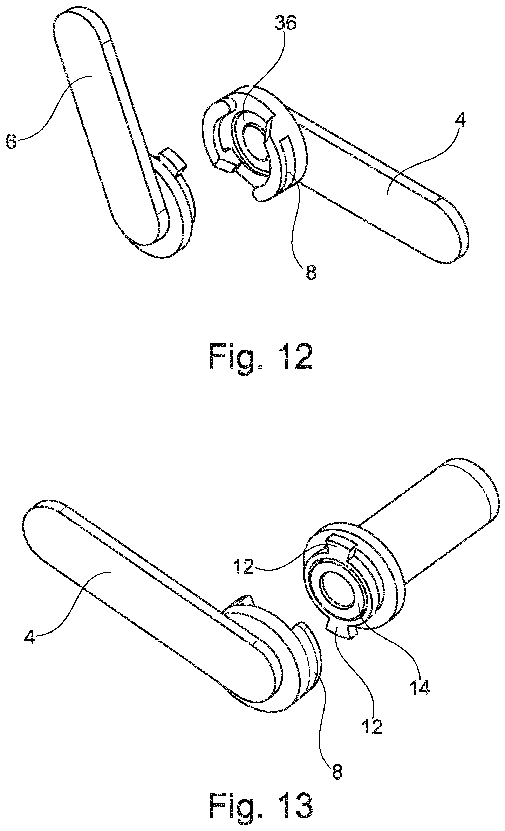

Die Figuren 12 bis 15 zeigen unterschiedliche Phasen beim Verbinden des ersten formstabilen Bauteils 4 mit dem zweiten formstabilen Bauteil 6. Das erste formstabile Bauteil 4 verfügt über ein erstes Magnetelement 36 in Form eines magnetischen Rings. Es verfügt zudem über das erste Formschlusselement 8 in Form eines umlaufenden Schlitzes. Das zweite formstabile Bauteil 6 weist das zweite Magnetelement 14 auf, das in Figur 12 nicht zu erkennen ist. Es ist ebenfalls in Form eines magnetischen Rings ausgebildet und korrespondiert zum ersten Magnetelement 36 des ersten formstabilen Bauteils 4. Das zweite Formschlusselement 12 ist in Form zweier Vorsprünge ausgebildet, von denen in Figur 12 nur einer zu erkennen ist. Zum Verbinden der beiden formstabilen Bauteile 4, 6 werden die beiden Bauteile 4, 6 einander angenähert, sodass zwischen den beiden Magnetelementen 36, 14 eine anziehende Kraft hervorgerufen wird.

Figur 13 zeigt die Situation aus einer anderen Perspektive. In dieser sind die beiden Vorsprünge des zweiten Formschlusselementes 12 sowie das zweite Magnetelement 14 gut zu erkennen.

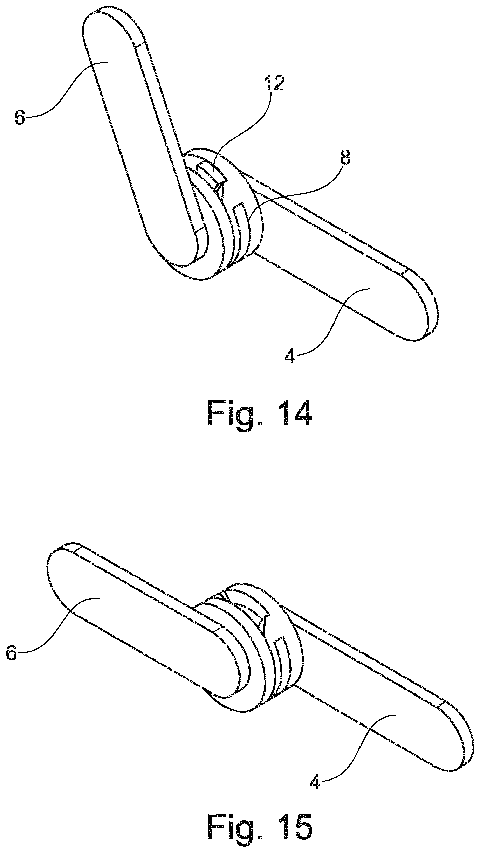

In der in Figur 14 gezeigten Position sind das erste Formschlusselement 8 und das zweite Formschlusselement 12so nah aneinander angeordnet, dass die Vorsprünge des zweiten Formschlusselementes 12 in den Schlitz des ersten Formschlusselementes eingreifen. Sie können dann nach Art eines Bajonett-Verschlusses gegeneinander verschoben werden, indem die beiden formstabilen Bauteile 4, 6 relativ zueinander verdreht werden. Diese Situation ist in Figur 15 dargestellt.

### Bezugszeichenliste

2 Orthese
4 erstes formstabiles Bauteil
6 zweites formstabiles Bauteil
8 erstes Formschlusselement
10 Ausnehmung
12 zweites Formschlusselement
14 zweites Magnetelement
16 Vorsprung
18 Nut
20 Stirnverzahnung
22 Innenseite
24 Außenseite
26 Einzelmagnete
28 Unterseite
30 Pfeil
32 Begrenzung
34 Oberseite
36 erstes Magnetelement

## Patentansprüche

1. Verfahren zum Anlegen einer Orthese (2) mit einem ersten formstabilen Bauteil (4), das ein erstes Magnetelement (36) und ein erstes Formschlusselement (8) aufweist, und einem zweiten formstabilen Bauteil (6), das ein zweites Magnetelement (14) und ein zweites Formschlusselement (12) aufweist, wobei das Verfahren folgende Schritte aufweist:
- Anlegen des ersten Bauteiles (4) an ein Körperteil des Trägers, so dass es sich entlang des Körperteiles erstreckt, wobei das erste Bauteil (4) noch nicht mit dem zweiten Bauteil (6) verbunden ist,
- Positionieren des zweiten Bauteiles (6) an dem ersten Bauteil (4),
wobei im angelegten Zustand der Orthese (2) zwischen dem ersten Magnetelement und dem zweiten Magnetelement eine magnetische Anziehungskraft in eine erste Richtung wirkt,
und das erste Formschlusselement mit dem zweiten Formschlusselement eine formschlüssige Verbindung bildet, durch die eine Bewegung der beiden Bauteile relativ zueinander verhindert wird,
wobei nach dem Positionieren des zweiten Bauteils (6) am ersten Bauteil (4) die formschlüssige Verbindung hervorgerufen wird, indem das erste Formschlusselement und das zweite Formschlusselement miteinander in Eingriff gebracht werden, wobei das erste Formschlusselement und/oder das zweite Formschlusselement relativ zueinander bewegt werden,
**dadurch gekennzeichnet, dass** das erste Formschlusselement und/oder das zweite Formschlusselement durch eine Kraft, die von dem ersten Magnetelement und/oder dem zweiten Magnetelement ausgeübt wird, aus einer ersten Position in eine zweite Position gebracht wird, wodurch die formschlüssige Verbindung hergestellt wird.

2. Verfahren nach Anspruch 1, **dadurch gekennzeichnet, dass** das zweite Bauteil (6) beim Positionieren an dem ersten Bauteil (4) zunächst dem ersten Bauteil (4) angenähert wird, so dass die anziehende Kraft entsteht, so dass die anziehende Kraft das zweite Bauteil (6) zumindest auch in die gewünschte Position zieht.

3. Verfahren nach einem der vorstehenden Ansprüche, **dadurch gekennzeichnet, dass** das erste Formschlusselement und/oder das zweite Formschlusselement durch ein Betätigungselement betätigbar ist und betätigt wird, um die formschlüssige Verbindung herzustellen.

4. Verfahren nach einem der vorstehenden Ansprüche, **dadurch gekennzeichnet, dass** die magnetische Anziehungskraft und die formschlüssige Verbindung beim Positionieren des zweiten Bauteils (6) am ersten Bauteil (4) entstehen.

5. Verfahren nach einem der vorstehenden Ansprüche, **dadurch gekennzeichnet, dass** das zweite Bauteil (6) beim Positionieren an dem ersten Bauteil (4) an einem zweiten Körperteil des Trägers angeordnet wird.

6. Verfahren nach einem der vorstehenden Ansprüche, **dadurch gekennzeichnet, dass** das erste Bauteil (4) in unterschiedlichen Positionen und/oder Orientierungen an dem zweiten Bauteil (6) befestigbar ist.

7. Orthese (2) mit einem ersten Bauteil (4), das ein erstes Magnetelement (36) und ein erstes Formschlusselement (8) aufweist, und einem zweiten Bauteil (6), das ein zweites Magnetelement (14) und ein zweites Formschlusselement (12) aufweist, wobei im angelegten Zustand der Orthese (2) zwischen dem ersten Magnetelement und dem zweiten Magnetelement eine magnetische Anziehungskraft in eine erste Richtung wirkt, und das erste Formschlusselement mit dem zweiten Formschlusselement eine formschlüssige Verbindung bildet, durch die eine Bewegung der beiden Bauteile relativ zueinander verhindert wird, wobei die Orthese derart ausgebildet ist, dass nach dem Positionieren des zweiten Bauteils (6) am ersten Bauteil (4) die formschlüssige Verbindung hervorgerufen werden kann, indem das erste Formschlusselement und das zweite Formschlusselement miteinander in Eingriff gebracht werden, wobei das erste Formschlusselement und/oder das zweite Formschlusselement relativ zueinander bewegt werden,
**dadurch gekennzeichnet, dass** die Orthese (2) eingerichtet ist, mit einem Verfahren gemäß einem der vorstehenden Ansprüche angelegt zu werden und dass die Orthese derart ausgebildet ist, dass nach dem Positionieren des zweiten Bauteils (6) am ersten Bauteil (4) das erste Formschlusselement und/oder das zweite Formschlusselement durch eine Kraft, die von dem ersten Magnetelement und/oder dem zweiten Magnetelement ausgeübt wird, aus einer ersten Position in eine zweite Position bringbar ist, wodurch die formschlüssige Verbindung hergestellt wird.

8. Orthese (2) nach Anspruch 7, **dadurch gekennzeichnet, dass** das erste Magnetelement und/oder das zweite Magnetelement mehrere Einzelmagnete (26) aufweist, die in unterschiedlichen Nord-Süd-Orientierungen an dem jeweiligen Bauteil angeordnet sind.

9. Orthese (2) nach Anspruch 8, **dadurch gekennzeichnet, dass** das erste und/oder das zweite Magnetelement wenigstens ein magnetisierbares Element aufweist, das im angelegten Zustand mit einem magnetischen Element des jeweils anderen Bauteils zusammenwirkt.

10. Orthese (2) nach Anspruch 7, 8 oder 9, **dadurch gekennzeichnet, dass** wenigstens ein Magnetelement oder wenigstens ein Einzelmagnet eines Magnetelementes an einem Formschlusselement befestigt ist.

11. Orthese (2) nach einem der Ansprüche 7 bis 10, **dadurch gekennzeichnet, dass** das erste Formschlusselement und/oder das zweite Formschlusselement durch ein Betätigungselement betätigbar ist, wobei das Betätigungselement vorzugsweise einen Magneten aufweist.

## Claims

1. A method for putting on an orthosis (2) with a first dimensionally stable component (4), comprising a first magnetic element (36) and a first form-fitting element (8), and a second dimensionally stable component (6), comprising a second magnetic element (14) and a second form-fitting element (12), the method comprising the following steps:
- putting the first component (4) against a part of the wearer's body so it extends along the body part, wherein the first component is not yet connected to the second component,
- positioning the second component (6) on the first component (4),
wherein a magnetic attractive force acts between the first magnetic element and the second magnetic element in a first direction when the orthosis (2) is mounted,
and the first form-fitting element forms a form-fitting connection with the second form-fitting element, by means of which a movement of the two components relative to each other is prevented,
wherein the form-fitting connection is created after positioning the second component (6) on the first component (4) by engaging the first form-fitting element and the second form-fitting element, wherein the first form-fitting element and/or the second form-fitting element are moved relative to each other,
**characterized in that** the first form-fitting element and/or the second form-fitting element is moved by a force exerted by the first magnetic element and/or the second magnetic element out of a first position into a second position, thus creating the form-fitting connection.

2. The method according to claim 1, **characterized in that**, during positioning on the first component (4), the second component (6) is initially moved closer to the first component (4), thereby generating an attraction force, so that the attraction force pulls the second component (6) at least also into the desired position.

3. The method according to one of the preceding claims, **characterized in that** the first form-fitting element and/or the second form-fitting element can be actuated by an actuation element and is actuated in order to create the form-fitting connection.

4. The method according to one of the preceding claims, **characterized in that** the magnetic attractive force and the form-fitting connection are generated during positioning of the second component (6) on the first component (4).

5. The method according to one of the preceding claims, **characterized in that** the second component (6) is arranged on a second part of the wearer's body during positioning on the first component (4).

6. The method according to one of the preceding claims, **characterized in that** the first component (4) can be fixed on the second component (6) in various positions and/or orientations.

7. An orthosis (2) with a first component (4), comprising a first magnetic element (36) and a first form-fitting element (8), and a second component (6), comprising a second magnetic element (14) and a second form-fitting element (12), wherein a magnetic attractive force acts between the first magnetic element and the second magnetic element in a first direction when the orthosis (2) is mounted, and the first form-fitting element forms a form-fitting connection with the second form-fitting element, by means of which a movement of the two components relative to each other is prevented wherein the orthosis is configured such that the form-fitting connection is created after positioning the second component (6) on the first component (4) by engaging the first form-fitting element and the second form-fitting element, wherein the first form-fitting element and/or the second form-fitting element are moved relative to each other **characterized in that** the orthosis is configured to be put on using a method according to one of the preceding claims and **in that** after positioning the second component on the first component the first form-fitting element and/or the second form-fitting element is moved by a force exerted by the first magnetic element and/or the second magnetic element out of a first position into a second position, thus creating the form-fitting connection

8. The orthosis (2) according to claim 7, **characterized in that** the first magnetic element and/or the second magnetic element have multiple individual magnets (26), which are arranged on the respective component in different north/south orientations.

9. The orthosis (2) according to claim 8, **characterized in that** the first and/or the second magnetic element has at least one magnetizable element which interacts with a magnetic element of the respective other component when mounted.

10. The orthosis (2) according to claim 7, 8 or 9, **characterized in that** at least one magnetic element or at least one individual magnet of a magnetic element is fixed on a form-fitting element.

11. The orthosis (2) according to one of the claims 7 to 10, **characterized in that** the first form-fitting element and/or the second form-fitting element can be actuated by an actuation element, the actuation element preferably comprising a magnet.

## Revendications

1. Procédé d'application d'une orthèse (2) comprenant un premier composant solide en forme (4) qui présente un premier élément magnétique (36) et un premier élément de liaison par complémentarité de forme (8), et comprenant un deuxième composant solide en forme (6) qui présente un deuxième élément magnétique (14) et un deuxième élément de liaison par complémentarité de forme (12), le procédé comprenant les étapes suivantes consistant à :
- appliquer le premier composant (4) contre une partie du corps du porteur de manière à ce qu'il s'étende le long de la partie du corps, le premier composant (4) n'étant pas encore relié au deuxième composant (6),
- positionner le deuxième composant (6) sur le premier composant (4),
dans lequel, à l'état appliqué de l'orthèse (2), une force d'attraction magnétique s'exerce entre le premier élément magnétique et le deuxième élément magnétique dans une première direction,
et le premier élément de liaison par complémentarité de forme établit une liaison par complémentarité de forme avec le deuxième élément de liaison par complémentarité de forme, laquelle empêche les deux composants de se déplacer l'un par rapport à l'autre,
après le positionnement du deuxième composant (6) sur le premier composant (4), la liaison par complémentarité de forme est établie du fait que le premier élément de liaison par complémentarité de forme et le deuxième élément de liaison par complémentarité de forme sont mis en prise l'un avec l'autre, le premier élément de liaison par complémentarité de forme et/ou le deuxième élément de liaison par complémentarité de forme étant déplacés l'un par rapport à l'autre,
**caractérisé en ce que** le premier élément de liaison par complémentarité de forme et/ou le deuxième élément de liaison par complémentarité de forme est déplacé d'une première position à une deuxième position par une force exercée par le premier élément magnétique et/ou par le deuxième élément magnétique, moyennant quoi la liaison par complémentarité de forme est établie.

2. Procédé selon la revendication 1,
**caractérisé en ce que** lors du positionnement sur le premier composant (4), le deuxième composant (6) est d'abord approché du premier composant (4), de sorte qu'une force d'attraction se produit, de sorte que la force d'attraction tire le deuxième composant (6) au moins également jusque dans la position souhaitée.

3. Procédé selon l'une les revendications précédentes,
**caractérisé en ce que** le premier élément de liaison par complémentarité de forme et/ou le deuxième élément de liaison par complémentarité de forme peut être actionné par un élément d'actionnement et est actionné pour établir la liaison par complémentarité de forme.

4. Procédé selon l'une des revendications précédentes,
**caractérisé en ce que** la force d'attraction magnétique et la liaison par complémentarité de forme se produisent lors du positionnement du deuxième composant (6) sur le premier composant (4).

5. Procédé selon l'une des revendications précédentes,
**caractérisé en ce que** lors du positionnement sur le premier composant (4), le deuxième composant (6) est agencé sur une deuxième partie du corps du porteur.

6. Procédé selon l'une des revendications précédentes,
**caractérisé en ce que** le premier composant (4) peut être fixé au deuxième composant (6) dans différentes positions et/ou orientations.

7. Orthèse (2) comprenant un premier composant (4) qui présente un premier élément magnétique (36) et un premier élément de liaison par complémentarité de forme (8), et comprenant un deuxième composant (6) qui présente un deuxième élément magnétique (14) et un deuxième élément de liaison par complémentarité de forme (12),
dans laquelle, à l'état appliqué de l'orthèse (2), une force d'attraction magnétique s'exerce entre le premier élément magnétique et le deuxième élément magnétique dans une première direction,
et le premier élément de liaison par complémentarité de forme établit une liaison par complémentarité de forme avec le deuxième élément de liaison par complémentarité de forme, laquelle empêche les deux composants de se déplacer l'un par rapport à l'autre,
l'orthèse est conçue de telle sorte que, après le positionnement du deuxième composant (6) sur le premier composant (4), la liaison par complémentarité de forme peut être provoquée du fait que le premier élément de liaison par complémentarité de forme et le deuxième élément de liaison par complémentarité de forme sont mis en prise l'un avec l'autre, le premier élément de liaison par complémentarité de forme et/ou le deuxième élément de liaison par complémentarité de forme étant déplacés l'un par rapport à l'autre,
**caractérisée en ce que** l'orthèse (2) est conçue pour être appliquée par un procédé selon l'une des revendications précédentes et **en ce que** l'orthèse est conçue de telle sorte qu'après le positionnement du deuxième composant (6) sur le premier composant (4), le premier élément de liaison par complémentarité de forme et/ou le deuxième élément de liaison par complémentarité de forme peut être déplacé d'une première position à une deuxième position par une force exercée par le premier élément magnétique et/ou le deuxième élément magnétique, moyennant quoi la liaison par complémentarité de forme est établie.

8. Orthèse (2) selon la revendication 7,
**caractérisée en ce que** le premier élément magnétique et/ou le deuxième élément magnétique comprend plusieurs aimants individuels (26) disposés selon différentes orientations nord-sud sur le composant respectif.

9. Orthèse (2) selon la revendication 8,
**caractérisée en ce que** le premier et/ou le deuxième élément magnétique comprend au moins un élément magnétisable qui, à l'état appliqué, interagit avec un élément magnétique de l'autre composant respectif.

10. Orthèse (2) selon la revendication 7, 8 ou 9,
**caractérisée en ce qu'**au moins un élément magnétique, ou au moins un aimant individuel d'un élément magnétique, est fixé à un élément de liaison par complémentarité de forme.

11. Orthèse (2) selon l'une des revendications 7 à 10,
**caractérisée en ce que** le premier élément de liaison par complémentarité de forme et/ou le deuxième élément de liaison par complémentarité de forme peut être actionné par un élément d'actionnement, l'élément d'actionnement comprenant de préférence un aimant.
